# EUROPEAN PATENT APPLICATION

(11) **EP 4 617 365 A1**
(43) Date of publication of application: **17.09.2025**
(21) Application number: 23899225.9
(22) Date of filing: 21.02.2023
(51) Int. Cl.: C12N 15/10, C12N 15/113, C12P 19/34, C12N 15/52, C12R 1/19

(54) **SYNTHESIS METHOD FOR DOUBLE-STRANDED RNA AND USE OF RNA LIGASE IN DOUBLE-STRANDED RNA SYNTHESIS**

(30) Priority: 08.12.2022 CN 202211574233
(71) Applicant: Asymchem Life Science (Tianjin) Co., Ltd, Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); LIU, Fang, Tianjin 300457 (CN); MA, Cuiping, Tianjin 300457 (CN); WANG, Zhaoshuai, Tianjin 300457 (CN); GENG, Yuhan, Tianjin 300457 (CN); CUI, Lixin, Tianjin 300457 (CN); ZHU, Wenxuan, Tianjin 300457 (CN); JIA, Xu, Tianjin 300457 (CN); YANG, Yiming, Tianjin 300457 (CN); LI, Juan, Tianjin 300457 (CN)
(74) Representative: Maiwald GmbH
(86) International application number: PCT/CN2023/077476
(87) International publication number: WO 2024/119625

(57) **Abstract**

Disclosed are a synthesis method for double-stranded RNA and use of an RNA ligase in double-stranded RNA synthesis. The synthesis method comprises the following steps: S1, synthesizing single-stranded RNA fragments; and S2, mixing the single-stranded RNA fragments and linking the single-stranded RNA fragments using the RNA ligase to form the double-stranded RNA. The RNA ligase is an RNA ligase derived from Vibrio phage, Escherichia phage, Klebsiella phage, or the like. By means of applying the technical scheme of the present invention, different nucleotide fragments can be specifically spliced, so that long-fragment nucleotide strands can be synthesized; meanwhile, the linkage of natural nucleotides and non-natural nucleotides can be catalyzed, thus solving the technical problems of synthesizing nucleotide strands at present.

## Description

### Technical Field

The present disclosure relates to the technical field of biology, and specifically relates to a synthesis method for double-stranded RNA, and use of an RNA ligase in the synthesis of double-stranded RNA.

### Background

Ribonucleic acid (RNA) is a kind of genetic information carrier existing in biological cells, and some of viruses and viroids. RNA has been only regarded as the molecule to transfer information between genes and proteins for a long time. In fact, RNA is the only biomolecule in the origin of life; it can not only store information, but also possesses the functions of enzymes. Besides acting as a messenger (mRNA) for protein synthesis, RNA further has a very important regulatory function. Non-transcription RNA includes miRNA, siRNA, IncRNA, piwiRNA, etc., of which there are more than 400 miRNA molecules to regulate and control at least one third of human genes. Since the 1970s, genetic vector technology, gene cloning technology, gene editing technology, and the like have brought profound effects on modern gene therapeutic technology. Meanwhile, RNA editing technology has achieved the editing of specific nucleotides in human cells at genetic levels. Such a technology can not only serve as a research tool, but also can be used as a temporary therapeutic method for the diseases caused by mutation.

In recent years, RNA has been developed rapidly in the field of medicines; on the one hand, small nucleic acid drugs represented by siRNA and ASO have yielded unusually brilliant results in rare diseases and other fields; on the other hand, mRNA drugs represented by mRNA vaccines have played an important role in COVID-19 pandemic. At the end of the last century, after the first small nucleic acid drug fomivirsen was approved and listed on the market, the correlated industry development has entered into a "window period". With the progress of delivery technologies, small nucleic acid drugs also have ushered in a rapid growth period in recent years. Moreover, the first blockbuster drug Nusinersen of small nucleic acid drugs has been incubated in the period. The drug is developed by Biogen and it is an antisense oligonucleotide (ASO) drug for the treatment of spinal muscular atrophy (SMA) in adult and child patients.

At present, the universal synthesis method for oligonucleotides (12-30 nucleotides) is mainly a solid-phase synthesis method. Solid-phase synthesis is to fix nucleic acids on a solid-phase carrier to complete a synthetic reaction. The most common solid phase carrier is the controlled pore glass (CPG), but its bearing capacity is limited and usually 70-80 µmol/g. However, there exist large shortcomings in the solid-phase synthesis of nucleotide chains; the synthetic chain length is limited; the technology is usually used to synthesize a chain length within 25 nucleotides. With the increase of the synthetic chain length, the yield reduces; when primers are synthesized to a chain length of about 80 nt, the crude product only has a purity of about 40%; hence, the synthesis of nucleotide chains is limited extremely.

Enzymatic synthesis is a green and efficient way. The synthesis way has relatively mild reaction conditions, demands for a low amount of organic reagent and even requires no organic reagent and thus, is a more promising catalysis way. A ligase can be utilized to synthesize a nucleotide chain to achieve green catalysis. In the meantime, its synthetic chain length is not limited, thus solving the technical bottleneck of solid-phase synthesis. However, there is almost no report on the catalyzed synthesis of natural or non-natural double-stranded RNA via an enzymic method in literatures or patents, particularly, the synthesis of double strands of non-natural RNA is hardly achieved.

### SUMMARY

The present disclosure is aimed at providing a synthesis method for double-stranded RNA, and use of an RNA ligase in the synthesis of double-stranded RNA, so as to solve the technical problem which is hardly solved by the catalyzed synthesis of natural or non-natural double-stranded RNA via enzymic methods in the prior art.

To achieve the aforesaid objective, one aspect of the present disclosure provides a synthesis method for double-stranded RNA. The synthesis method includes the following steps: S1, synthesizing single-stranded RNA fragments; S2, mixing the single-stranded RNA fragments, and linking the single-stranded RNA fragments with an RNA ligase to form the double-stranded RNA; the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1, or Buttiauxella phage vB_ButM_GuL6.*

Further, the single-stranded RNA fragments have a length of 3 to 200 nucleotides or analogues thereof.

Further, the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.

Further, the double-stranded RNA is natural RNA or non-natural RNA.

Further, the non-natural RNA has a ribonucleotide with one or more of pentose ring 2'-position modification and 3'-position modification, phosphate α-position modification or base modification; preferably, the pentose ring 2'-position modification includes 2'-methoxy modification, 2'-fluoro modification, 2'-trifluoromethoxy modification, 2'-methoxyethyl modification, 2'-allyl modification, 2'-amino modification, or 2'-azido modification; preferably, the phosphate α-position modification includes phosphate α-position sulfur modification; and preferably, the base modification includes methylation modification and/or acetylation modification at any one or more of N1, N5, or N6 positions of a base.

Further, the single-stranded RNA fragments are synthesized using a solid-phase synthesis method.

Further, the single-stranded RNA fragments include substrate 1 - substrate n of the single-stranded RNA fragments and substrate n+1 - substrate n+n of corresponding complementary single-stranded RNA fragments, where n ≥ 2, there are complementary base pairs of ≥ 3 nucleotides between the complementary single-stranded RNA fragments; there are base complementary pairs of ≥ 2 nucleotides at the 5'-ends of the substrate 2 and the substrate n+1, and the substrate n and the substrate n+n-1; optionally, 5'- and 3'-ends outside the substrate 1 and the substrate n+1 are linked by chemical bonds; and optionally, 5'- and 3'-ends outside the substrate n and the substrate n+n are linked by chemical bonds.

Further, the reaction temperature of S2 is 0°C to 60°C, preferably 4°C to 37°C, the reaction time is 0.5 h to 24 h, and the pH is 6 to 8.5.

Another aspect of the present disclosure provides use of an RNA ligase in the synthesis of double-stranded RNA, where the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1, or Buttiauxella phage vB_ButM_GuL6.*

Further, the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.

The technical solution of the present disclosure can be applied to specifically splice different nucleotide fragments, synthesize long-fragment nucleotide chains, and to catalyze the ligation between natural nucleotides and non-natural nucleotides, thereby solving the technical problem in the synthesis of nucleotide chains currently.

### Brief Description of the Drawings

Accompanying drawings of the description which constitute a part of the present application are provided to further understand the present disclosure; schematic examples of the present disclosure and its specification are used to interpret the present disclosure, but are not construed as limiting the present disclosure inappropriately. In the drawings:
FIG. 1 is a graphical result showing denaturing polyacrylamide gel electrophoresis in Example 1;
FIG. 2 is an ultra-performance liquid chromatography (UPLC) diagram of enzyme-free reaction control in Example 1;
FIG. 3 is a UPLC diagram of lig20-catalyzed reaction in Example 1;
FIG. 4 is a graphical result showing denaturing polyacrylamide gel electrophoresis in Example 2;
FIG. 5 is a graphical result showing denaturing polyacrylamide gel electrophoresis in Example 2;
FIG. 6 is a UPLC diagram of enzyme-free reaction control in Example 2;
FIG. 7 is a UPLC diagram of lig24-catalyzed reaction in Example 2;
FIG. 8 shows appearance time of a positive-sense strand product in Example 2;
FIG. 9 shows appearance time of an antisense strand product in Example 2;
FIG. 10 shows appearance time of a double-stranded product in Example 2;
FIG. 11 is a detection reaction result of sodium dodecyl sulfate-polyacrylamide gel electrophoresis (SDS-PAGE) diagram in Example 3;
FIG. 12 is a detection reaction result of SDS-PAGE diagram in Example 4;
FIG. 13 is a UPLC diagram in Example 4; and
FIG. 14 shows a mass spectrometry result in Example 4.

### Detailed Description of the Embodiments

It needs to be specified that examples and features in the examples of the present application may be combined with each other in case of no conflict. The present disclosure will be specified with reference to the accompanying drawings in combination with the examples below.

### Explanation of nouns

Natural RNA: refers to an RNA chain formed by linking natural nucleotides via phosphate ester linkages, its 2' position is hydroxyl and phosphate skeleton is a phosphorus-oxygen bond.

Non-natural RNA: refers to an RNA chain formed by linking non-natural nucleotides via phosphate ester linkages, its 2' position may be a modified one, e.g., 2'F, 2' methoxy, 2'MOE, LNA, etc., and also may be modified on bases, modified on a phosphate group, e.g., phosphorus-sulfur modification.

Currently, there is almost no report on the catalyzed synthesis of natural or non-natural double-stranded RNA via an enzymic method in literatures or patents, particularly, the synthesis of double strands of non-natural RNA is hardly achieved. A ligase is utilized in the present disclosure to synthesize natural or non-natural RNA, thus filling up the technical blank.

According to a typical embodiment of the present disclosure, provided is a synthesis method for double-stranded RNA. The synthesis method includes the following steps: S1, synthesizing single-stranded RNA fragments; S2, mixing the single-stranded RNA fragments, and linking the single-stranded RNA fragments by means of an RNA ligase so as to form the double-stranded RNA; the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1, or Buttiauxella phage vB_ButM_GuL6.*

The technical solution of the present disclosure can be applied to specifically splice different nucleotide fragments, synthesize long-fragment nucleotide chains, and to catalyze the ligation between natural nucleotides and non-natural nucleotides, thereby solving the technical problem in the synthesis of nucleotide chains currently.

In an embodiment of the present disclosure, the single-stranded RNA fragments have a length of 3 to 200 nucleotides or analogues thereof, and preferably 3 to 20 nucleotides or analogues thereof.

Preferably, the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.

According to a typical embodiment of the present disclosure, the double-stranded RNA is natural RNA or non-natural RNA. The non-natural RNA may contain 2' methoxy, 2' fluorine, 2' MOE, 2' amino, 2' methyl, FANA, LNA, or PS, where 2' methoxy, 2' fluorine, and PS have a better effect.

In the present disclosure, the single-stranded RNA fragments may be synthesized using a solid- -phase synthesis method, i.e., a synthesis mode in the prior art.

According to a typical embodiment of the present disclosure, the single-stranded RNA fragments include substrate 1 - substrate n of the single-stranded RNA fragments and substrate n+1 - substrate n+n of corresponding complementary single-stranded RNA fragments, where n ≥ 2, there are complementary base pairs of ≥ 3 nucleotides between the complementary single-stranded RNA fragments; there are base complementary pairs of ≥ 2 nucleotides at the 5'-ends of the substrate 2 and the substrate n+1, and the substrate n and the substrate n+n-1; optionally, 5'- and 3'-ends outside (outside is namely, the non-linked side) the substrate 1 and the substrate n+1 are linked by chemical bonds; and optionally, 5'- and 3'-ends outside (outside is namely, the non-linked side) the substrate n and the substrate n+n are linked by chemical bonds. Specifically, it may be understood in combination with the following reaction formula:

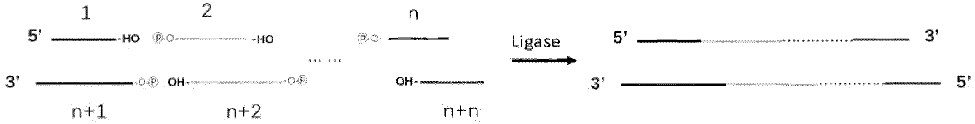

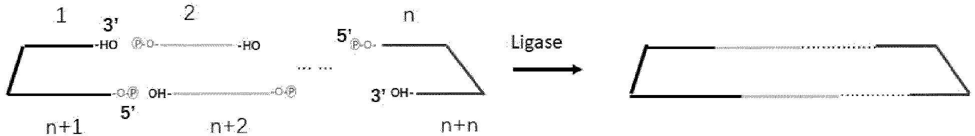

Preferably, the reaction temperature of S2 is 0°C to 60°C, more preferably 4°C to 37°C, the reaction time is 0.5 h to 24 h, and the pH is 6 to 8.5.

According to a typical embodiment of the present disclosure, provided is use of an RNA ligase in the synthesis of double-stranded RNA, where the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1, or Buttiauxella phage vB_ButM_GuL6.* These ligases may be adopted directly for reaction ligation without annealing reaction, thus providing synthetic efficiency. Preferably, the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.

The advantageous effects of the present disclosure will be further described in combination with the examples below.

To catalyze the double-strand ligation of short chains RNA or non-natural short chains RNA, the inventor excavated multiple different species/genus of genes from a gene pool, and cloned these genes into *Escherichia coli;* an expression vector was Pet28A; the constructed bacterium containing genes of interest were subjected to IPTG induced expression, cultured, and centrifuged to collect bacterial cells; the bacterial cells were ultrasonically crushed and centrifuged to obtain supernatant. The supernatant was purified with a nickel column in combination with purification with an ion column or a molecular sieve to obtain a pure enzyme.

0.1 µmol (a final concentration of 200 µM) of each sequence fragment, 0.1 mg of a ligase, 0.4 µmol of ATP, 0.5 µmol of DTT, and 5 µmol of MgCl₂ were added to a clean reactor, and supplemented with Tris-Cl buffer solution to 500 µL such that the final concentration of Tris-Cl was 50 mM; the reaction system was mixed and incubated at 25°C for 1-20 h; the obtained reaction solution was detected by polyacrylamide gel electrophoresis or UPLC to obtain the following results as shown in Table 3. The results show that most of enzymes have no linking activity, and only a small number of enzymes have catalytic activity.

DsRNA activity-1 is the activity of the following reaction: 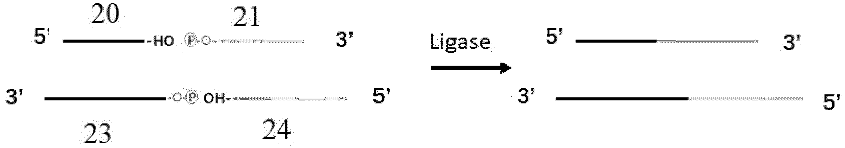 where the specific sequence is shown in Table 1.

**Table 1**

| No. (SEQ ID NO.) | Sequence | 5' end | 3' end |
|---|---|---|---|
| 20 | UmGmAfGfUmGm | Hydroxy | Hydroxy |
| 21 | AfGfAmCmCfUfGmCm | Phosphate group | Hydroxy |
| 23 | UfCmUmCfAfCmUmCfAfCmUm | Phosphate group | Hydroxy |
| 24 | AmGfUfCmGmCfAfGmGm | Hydroxy | Hydroxy |

DsRNA activity-2 is the activity of the following reaction: 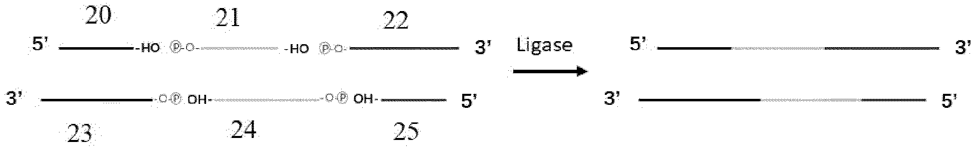 where the specific sequence is shown in Table 2.

**Table 2**

| No. (SEQ ID NO.) | Sequence | 5' end | 3' end |
|---|---|---|---|
| 20 | UmGmAfGfUmGm | Hydroxy | Hydroxy |
| 21 | AfGfAmCmCfUfGmCm | Phosphate group | Hydroxy |
| 22 | GmAmCfUfGmCmAfCfUmAm | Phosphate group | Hydroxy |
| 23 | UfCmUmCfAfCmUmCfAfCmUm | Phosphate group | Hydroxy |
| 24 | AmGfUfCmGmCfAfGmGm | Phosphate group | Hydroxy |
| 25 | AmGfUfGmCm | Hydroxy | Hydroxy |

**Table 3**

| *S.N. in the patent* | Species/genus | Gene bank No. | dsRNA activity-1 | dsRNA activity-2 |
|---|---|---|---|---|
| lig-1 | Aeropyrum pernix | WP_010866453.1 | - | - |
| lig-2 | Cavia porcellus | XP_003477632.1 | - | - |
| lig-3 | Escherichia coli | CAD6000712.1 | - | - |
| lig-4 | Mycobacterium tuberculosis | CNW30100.1 | - | - |
| lig-5 | Candida albicans | AAA34373.2 | - | - |
| lig-6 | Methanopyrus kandleri | WP_011019294.1 | - | - |
| lig-7 | *Chlorella virus* | 2Q2T_A | - | - |
| lig-8 | *Chlorella virus* | Lig-7 mutant | - | - |
| lig-9 | *Trypanosoma brucei gambiense* | XP_011771454.1 | - | - |
| lig-10 | *Diplonema papillatum* | ALJ92478.1 | - | - |
| lig-11 | *virus* | NP_054135.1 | - | - |
| lig-12 | *fungus Neurospora crassa* | XP_011393315.1 | - | - |
| lig-13 | *fungus Magnaporthe grisea* | XP_003712962.1 | - | - |
| lig-14 | *Archaea* | 4DWR_A | - | - |
| lig-15 | *Bacteriophage RB69* | NP_861926.1 | - | - |
| lig-16 | Aeromonas virus Aeh1 | NP_944126.1 | - | - |
| lig-17 | Citrobacter phage Merlin | YP_009203914.1 | - | - |
| lig-18 | *Trypanosoma brucei brucei* | P86926.1 | - | - |
| lig-19 | *Trypanosoma brucei brucei* | XP_011771454.1 | - | - |
| lig-20 | *Vibrio phage* | NP_899381.1 | ++++ | ++++ |
| lig-21 | *Vibrio phage* | YP_009046856.1 | ++ | ++ |
| lig-22 | *Vibrio phage* | YP_009006379.1 | +++ | ++ |
| lig-23 | *Escherichia phage* | QHR70627.1 | ++ | ++ |
| lig-24 | *Klebsiella phage* | YP_007348874.1 | +++ | +++ |
| lig-25 | *Vibrio phage* | QNI20667.1 | ++++ | ++++ |
| lig-26 | *Acidobacteriabacterium* | MBF84567.1 | + | + |
| lig-27 | *Salmonellaenterica* | EBJ7676734.1 | +++ | ++ |
| lig-28 | *YersiniaphagevB_YepM_ZN18* | YP_010077676.1 | + | + |
| lig-29 | *Shigella phage pSs-1* | YP_009110995.1 | ++ | + |
| lig-30 | *Buttiauxella phage vB_ButM_GuL6* | QJI10731.1 | + | + |
| lig-31 | *Bacteriophage AR1* | WP_179226752.1 | ++ | ++ |
| lig-32 | *Vibrio phage* | QHJ74267.1 | ++ | ++ |
| lig-33 | *Vibrio phage phi-ST2* | YP_009006379.1 | +++ | ++ |
| lig-34 | *Escherichia phage JN02* | QNI20694.1 | ++ | +++ |
| lig-35 | *Klebsiella phage KP15* | YP_003580083.1 | +++ | ++ |
| lig-36 | *Vibrio phage JS98* | YP_001595305.1 | ++ | + |
| lig-37 | *Rhodothermus phage RM378* | NP_835690.1 | + | + |
| lig-38 | *Vibrio phage phi-ST2* | YP_009006096.1 | ++ | + |

| | | | | |
|---|---|---|---|---|
| Note: "-" denotes: no product generation; "++" denotes: a conversion rate of greater than 10% and less than 50%; "+++" denotes: a conversion rate of greater than 50% and less than 70%; "++++" denotes: a conversion rate of greater than 70%. | | | | |

Amino acid sequences of the partial enzymes are set forth below:
lig-20 (SEQ ID NO.6, *Vibrio phage*): 
MSFVKYTSLENSYRQAFVDKCDMLGVREWVALEKIHGANFSFIVEFKPNEAQDGAEFT
VTPAKRTSTIGANVMGDYDFYGCTSVVEAHTAKMEAISNWLWARGIINVGETIIVYGELAGKG
VQKEVNYGDKDFWAYDILLPETGKFLDWDVVLEACEFAKVKTTHEIARGTLDELLRIDPLFRS
FHTPADVDGDNVAEGFVVKQLRNEKRLHNGSRAILKVKNDKFKEKKNKAGKTPRAAVVLTE
EQEKLHAAFSCYLTENRLRNVLSKLETVTQKQFGMISGLFIKDAKDEFERDELNETAIARDD
 WDVIKRSLTNIANEILRKNWLDILDGNF
lig-21 (SEQ ID NO.1, *Vibrio phage*): 
MSFVKYTSLENSYRQAFVDKCDMLGVRDWVALEKIHGANFSFIVEFDGGYTVTPAKRT
SIIGATATGDYDFYGCTSVVEAHKEKVELVANFLWLNEYINLYEPIIIYGELAGKGIQKEVNYGD
KDFWAFDIFLPQREEFVDWDTCVAAFTNAEIKYTKELARGTLDELLRIDPLFKSLHTPAEHEG
DNVAEGFVVKQLHSEKRLQSGSRAILKVKNEKFKEKKKKEGKTPTKLVLTPEQEKLHAEFSC
YLTENRLKNVLSKLGTVNQKQFGMISGLFVKDAKDEFERDELNEVAIDRDDWNAIRRSLTNIA
NEILRKNWLNILDGNF
lig-22 (SEQ ID NO.2, *Vibrio phage*): 
MNVQELYKNLMSLADDAEGKFFFADHLSPLGEKFRVFSYHIASYSDWLLPGALEARGIM
FQLDDNDEMIRIVSRPMEKFFNLNENPFTMELDLTTTVQLMDKADGSLISTYLSGENFALKSK
TSIFSEQAVAANRYIKKPENRDLWEFCDDCTQAGLTVNMEWCAPNNRIVLEYPEAKLVILNIR
DNETGDYVSFDDIPQSALMRVKQWLVDEYDPATAHEPDFVEKLRDTKGIEGMILRLANGQSV
KIKTQWYVDLHSQKDSVNVPKKLVTTILNGNHDDLYALFADDKPTIERIREFDSHVTKTLTNSF
NAVRQFYARNRHLARKDYAIAGQKVLKPWEFGVAMIAYQKQTVEGVYESLVTAYLKRPELAIP
EKYLNGV
lig-23 (SEQ ID NO.3, *Escherichia phage*): 
MFKKYSSLENHYNSKFIEKLRTNGLTGGEWVAREKIHGTNFSLIIERDAVTCAKRTGPILP
AEDFYGYEIVLKNYADSIKSIQDIMETSAAVSYQVFGEFAGTGIQKNVDYGDKDFYVFDIIVTT
ESGDVTYVDDYMMESFCKTFKFKMAPLLGRGKFEDLIKLPNDLDSVLPDYNFTVDNVGLAE
ANAHVWNAEAKGEVFTAEGYVLKPCYPLWLPNGNRVAIKCKNSKFSEKKKTDKPIKVAVVLS
QDDLDLLQQFTDYVTVNRINNVISKIGEVSPKDFGKVMGLTVQDILEEAAREELELTDAENPV
EVKKQLIECVKDTLRAVWIELVSK
lig-24 (SEQ ID NO.4, *Klebsiella phage*): 
MFKKYSSLTNHYEGKFINGVIMNGLTGGVWVAREKIHGANFSFITDDGITVTPAKRTDVV
KPAEDFYGCSAVVAKYSPGIRKMWETLKKTGTYDDLVIQVYGEFAGRGVQKDVDYGEKDFY
VFDIRVNGEFLPDNLCSLISRSHGLKMAPLLGYGTFEEIKELPITFESVVNKANSGIGSDNTVY
GEFVYPIMDVEEGNIAEGFVMKPVSPAFMPNGERVAIKCKTTKFTEKKAKKATRFNAPVSLS
EKDKNQLDEFVCYLTENRVKNVLSKLDLASITAKDFGRIMGLTVQDAIEEISRNHGPFLEQFE
DPAMAKKLFVTEAQNMIRPVWGKILNHEF
lig-25 (SEQ ID NO.5, *Vibrio phage*): 
MFKKYSSLENHYNSKFIEKLYTNGLTTGVWAREKIHGTNFSLIIERDNVTCAKRTGPILP
AEDFYGYEIVLKKYDKAIKAVQEVMESISTSVPVSYQVFGEFAGGGIQKGVDYGEKDFYVFDI
IINTESDDTYYMSDYEMQDFCNTFGFKMAPMLGRGTFDSLIMIPNDLDSVLAAYNSTASEDLV
 
EANNCVFDANVIGDNTAEGYVLKPCFPKWLSNGTRVAIKCKNSKFSEKKKSDKPVKTQVPLT
EIDKNLLDVLACYVTLNRVNNVISKIGTVTPKDFGKVMGLTVQDILEETSREGIVLTSSDNPNL
VKKELVRMVQDVLRPAWIELVS。
lig-26 (SEQ ID NO.7, AbRnl, derived from *Acidobacteriabacterium,* Rnl1 family): 
MKQMYDNLKALTQKNDMFFSSIQTTPSNKPVEIFSYHIASYSDWLEPDAIECRGIMFDIT
DKENPVILSRPMEKFFNLGENPLALPEDVLPLVKTIEEKRDGSLISTYLDDGKLYTKSKGSLYS
DQANDSNRFLMRSENKDFKQALKTLAELGYTANMEYTSPKNQIVLKYEKQELIVLNIRNTET
GDYLTLDDLAELDAENPEHTIYTHIAERFVDYEEVDGMSDEDVERIRDMKGIEGYVLVGQNQ
RVKLKTDWYVNLHRLKDNINNNRRLVESVVESRTDDLRQLFEHDQGSIDKIKEFEQYVLSKIK
SCLAEVRTVYERVKHLDRKHYAINAQRYVDYTPLFSVIMRQFDDYNEENVVENIKQIALKNVD
DFIPMHYK。
lig-27 (SEQ ID NO.8, SeRnl, derived from *Salmonellaenterica,* Rnl2 family): 
MFKKYSSLENHYNSKFIEKLYSLGLTGGEWVAREKIHGTNFSLIIERDKVTCAKRTGPILP
AEDFFGYEIILKNYADSIKAVQDIMETSAVVTYQVFGEFAGPGIQKNVDYGDKDFYVFDIIVTTE
SGDVTYVDDYMMESFCNTFKFKMAPLLGRGKFEELIKLPNDLDSVVQDYNFTVDHAGLVDA
NKCVWNAEAKGEVFTAEGYVLKPCYPSWLPNRNRVAIKCKNSKFSEKKKSDKPIKTKVELSE
ADNKLMGILACYVTLNRVNNVISKIGEIGPKDFGKVMGLTVQDILEETSREGITLTQADNPSLIK
KELVKMVQDVLRPAWIELVS。
lig-28 (SEQ ID NO.9, YpRnl, derived from *YersiniaphagevB_YepM_ZN18,* Rnl2 family): 
MFKKYSSLENHYNSKFIEKLYSLGLTGGEWVAREKIHGTNFSLIIERDKVTCAKRTGPILP
AEDFFGYEIILKNYEDSIKAVQDIMETSAVVSYQVFGEFAGPGIQKNVDYGDKDFYVFDIIVTT
ESGDVTYVDDYMMESFCNTFKFKMAPLLGRGKFEELIKLPNDLDSVVQDYNFTVDHAGLVD
ANKCVWKAEAKGEVFTAEGYVLKPCYPSWLHNGNRVAIKCKNSKFSEKKKSDKPIKAKVEL
SEADNKLVGILACYVTLNRVNNVISKIGEIGPKDFGKVMGLTVQDILEETSREGITLTQADNPS
LVKKELVKMVQDVLRPAWIELVS。
lig-29 (SEQ ID NO.10, SppRnl, derived from *Shigella phage pSs-1,* Rnl2 family): 
MFKKYSSLENHYNSKFIEKLYSLGLTGGEWVAREKIHGTNFSLIIERDKVTCAKRTGPILP
AEDFFGYEIILKNYADSIKAVQDIMETSAVVSYQVFGEFAGPGIQKNVDYGDKDFYVFDIIVTT
ESGDVTYVDDYMMESFCNTFKFKMAPLLGRGKFEELIKLPNDLDSVVQDYNFTVDHAGLVD
ANKCVWNAEAKCEVFTAEGYVLKPCYPSWLPNRNRVAIKCKNSKFSEKKKSDKPIKAKVELS
EADNKLVGILACYVTLNRVNNVISKIGEIGPKDFGKVMGLTVQDILEETSREGITLTQADSPSL
VKKELVKMVQDVLRPAWIELVS。
lig-30 (SEQ ID NO.11, BpRnl, derived from *Buttiauxella phage vB_ButM_GuL6,* Rnl2 family): 
MFVKYSSLTNHYEGKFINGVIMNGLTGGVWVAREKIHGANFSLITSDGEKVIPAKRSGEI
LPTEQFYGCEPVVARYAPAIRKLWDVINTAQQVSGIYVDSLVIQVYGEFAGRGVQKDVDYGE
KDFYVFDIRVNGEFLHDNIVAVYAKSVGLKMAPLLAYGTFDEIRALPITFDSVVNLANSGAIPA
HNGVEPEFKNFMTLKDGEGENIAEGFVMKPVHPAFMPNGERVAIKCKTTKFSEKKNKQANR
FNAPAELSENDKAKLDVFTCYLTENRVKNVLSKIDSANLTAKDFGRVMGLTVQDALEEIERNY
GPFIEQFENPTLAKKTFTNEASSLIRTNWGAILNNEF
lig-31 (SEQ ID NO.12, BpARnl, derived from *Bacteriophage AR1,* Rnl1 family): 
MQELFNNLMELCKDSQRKFFYSDDVSASGRTYRIFSYNYASYSDWLLPDALECRGIMF
EMDGEKPVRIASRPMEKFFNLNENPFTMNIDLNDVDYILTKEDGSLVSTYLDGDEILFKSKGS
IKSEQALMANGILMNINHHQLRDRLKELAEDGFTANFEFVAPTNRIVLAYQEMKIILLNIRENET
GEYISYDDIYKDAALRPYLVERYEIDSPKWVEEAKNAENIEGYVAVMKDGSHFKIKSDWYVSL
HSTKSSLDNPEKLFKTIIDGASDDLKAMYADDEYSYRKIEAFETTYLKYLDRALFLVLDCHNK
HCGKDRKTYAMEAQGVAKGAGMDHLFGIIMSLYQGYDSQEKVMCEIEQNFLKNYKKFIPEG
Y
lig-32 (SEQ ID NO.13, VPhRnl, derived from *Vibrio phage,* Rnl1 family): 
MTTQELYNHLMTLTDDAEGKFFFADHISPLGEKLRVFSYHIASYSDWLLPGALEARGIMF
QLDEQDKMVRIVSRPMEKFFNLNENPFTMDLDLTTTVQLMDKADGSLISTYLTGENFALKSK
TSIFSEQAVAANRYIKLPENRDLWEFCDDLTQAGCTVNMEWCAPNNRIVLEYPEAKLVILNIR
DNETGDYVSFDDIPLPALMRVKKWLVDEYDPETAHADDFVEKLRATKGIEGMILRLANGQSV
KIKTQWYVDLHSQKDSVNVPKKLVTTILNNNHDDLYALFADDKPTIDRIREFDSHVSKTVSAS
FHAVSQFYVKNRHMSRKDYAIAGQKTLKPWEFGVAMIAYQNQTVEGVYEALVGAYLKRPELL
IPEKYLNEA
lig-33 (SEQ ID NO.14, VPpRnl, derived from *Vibrio phage phi-ST2,* Rnl2 family): 
MNVQELYKNLMSLADDAEGKFFFADHLSPLGEKFRVFSYHIASYSDWLLPGALEARGIM
FQLDDNDEMIRIVSRPMEKFFNLNENPFTMELDLTTTVQLMDKADGSLISTYLSGENFALKSK
TSIFSEQAVAANRYIKKPENRDLWEFCDDCTQAGLTVNMEWCAPNNRIVLEYPEAKLVILNIR
DNETGDYVSFDDIPQSALMRVKQWLVDEYDPATAHEPDFVEKLRDTKGIEGMILRLANGQSV
KIKTQWYVDLHSQKDSVNVPKKLVTTILNGNHDDLYALFADDKPTIERIREFDSHVTKTLTNSF
NAVRQFYARNRHLARKDYAIAGQKVLKPWEFGVAMIAYQKQTVEGVYESLVTAYLKRPELAIP
EKYLNGV
lig-34 (SEQ ID NO.15, EPRnl, derived from *Escherichia phage JN02,* Rnl1 family): 
MEKLYYNLLSLCKSSSDRKFFYSDDVSPIGKKYRIFSYNFASYSDWLLPDALECRGIMFE
MDGETPVRIASRPMEKFFNLNENPFTLSINLDDVKYLMTKEDGSLVSTYLDGGTVRFKSKGS
IKSDQAVSATSILLDIDHKNLADRLLELCNDGFTANFEYVAPTNKIVLTYPEKRLILLNIRDNNTG
 
EYIEYDDIYLDPVFRKYLVDRFEVPEGDWTSDVKSSTNIEGYVAVMKDGSHFKLKTDWYVAL
HTTRDSISSPEKLFLAIVNGASDDLKAMYADDEFSFKKVELFEKAYLDFLDRSFYICLDTYDKH
KGKDRKTYAIEAQAVCKGAQTPWLFGIIMNLYQGGSKEQMMTALESVFIKNHKNFIPEGY
lig-35 (SEQ ID NO.16, KPRnl, derived from *Klebsiella phage KP15,* Rnl2 family): 
MFKKYSSLTNHYEGKFINGVIMNGLTGGVWVAREKIHGANFSFITDDGITVTPAKRTDVV
KPAEDFYGCSAVVAKYSPGIRKMWETLKKTGTYDDLVIQVYGEFAGRGVQKDVDYGEKDFY
VFDIRVNGEFLPDNLCSLISRSHGLKMAPLLGYGTFEEIKELPITFESVVNKANSGIGSDNTVY
GEFVYPIMDVEEGNIAEGFVMKPVSPAFMPNGERVAIKCKTTKFTEKKAKKATRFNAPVSLS
EKDKNQLDEFVCYLTENRVKNVLSKLDLASITAKDFGRIMGLTVQDAIEEISRNHGPFIEQFED
PAMAKKLFVTEAQNMIRPVWGKILNHEF
lig-36 (SEQ ID NO.17, VPJRnl, derived from *Vibrio phage JS98,* Rnl2 family): 
MFKKYSSLENHYNSKFIEKLRTNGLTGGEWVAREKIHGTNFSLIIERDAVTCAKRTGPILP
AEDFYGYEIVLKNYADSIKSVQHLIESINYQSYQIYGELAGPGIQKNVDYGDKDFYVFDIRVTK
EDGTESVLTDTLMEAFCIIHKFKVAPCLATGSFEDLIKLPNDFDSVIPDYNFAVDNAGLTIANST
DFIPKVEGKVFTAEGFVLKPDIPTWLPNGNRVAIKCKNSKFSEKKKSDKPIKAAVVLSQDDMD
LMWQFTDYVTVNRINNVISKIGEVSKKDFGKVMGLTVQDILEEAAREELELTDAENPVEVKK
QLVECVKDTLRAVWIELVS
lig-37 (SEQ ID NO.18, TBRnl, derived from *thermophilic bacteriophage RM378,* Rnl1 family): 
MESMNVKYPVEYLIEHLNSFESPEVAVESLRKEGIMCKNRGDLYMFKYHLGCKFDKIYH
LACRGAILRKTDSGWKVLSYPFDKFFNWGEELQPEIVNYYQTLRYASPLNEKRKAGFMFKL
PMKLVEKLDGTCVVLYYDEGWKIHTLGSIDANGSIVKNGMVTTHMDKTYRELFWETFEKKYP
PYLLYHLNSSYCYIFEMVHPDARVVVPYEEPNIILIGVRSVDPEKGYFEVGPSEEAVRIFNESG
GKINLKLPAVLSQEQNYTLFRANRLQELFEEVTPLFKSLRDGYEVVYEGFVAVQEIAPRVYYR
TKIKHPVYLELHRIKTTITPEKLADLFLENKLDDFVLTPDEQETVMKLKEIYTDMRNQLESSFD
TIYKEISEQVSPEENPGEFRKRFALRLMDYHDKSWFFARLDGDEEKMQKSEKKLLTERIEKG
LFK
lig-38 (SEQ ID NO.19, VPphRnl, derived from *Vibrio phage phi-ST2,* Rnl1 family):

MSFVKYTSLENSYRQAFVDKCDMLGVKEWVALEKIHGANFSFIVEFKPSTEEVPGEMS
VTPAKRTSTIGANAMGDYDFYGCTSVVEAHIEKMQDISNWLFANDFIKNDETIIVYGELAGKGI
QKEVNYGDKDFWAYDILCPETGEFLDWDVVLKACKFAGVKTTHEIARGTLDELLKIDPLFRSF
HTPADVDSENVAEGFVVKQLKAEKRLHNGSRAILKVKNEKFKEKKNKQGKTPRAKVVLTEE
QEKLHAAFSCYLTENRLRNVLSKIGKVEAKQFGMVSGLFVKDAKDEFERDERDEVAIPRDD
WDVIKRSLVNVANEILRKNWLNIVDGTF

### Example 1

### Preparation of a ligase

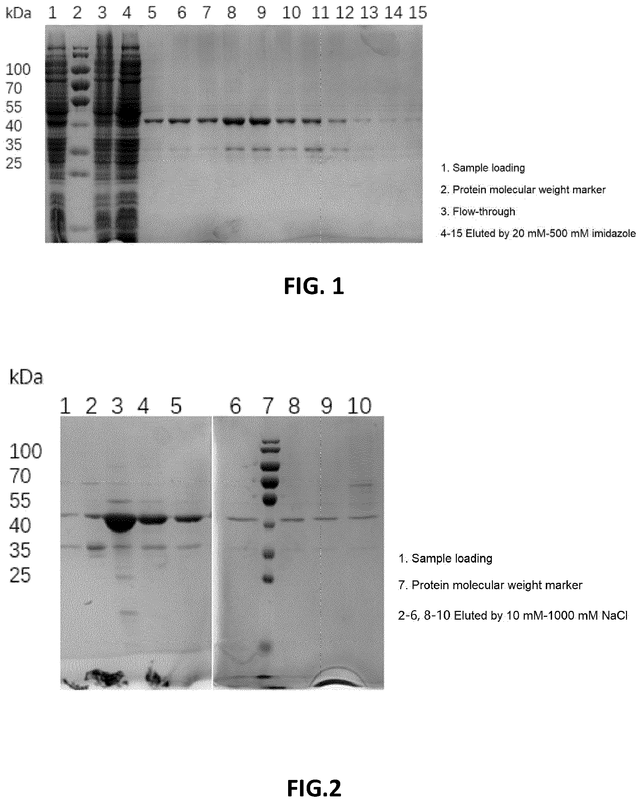

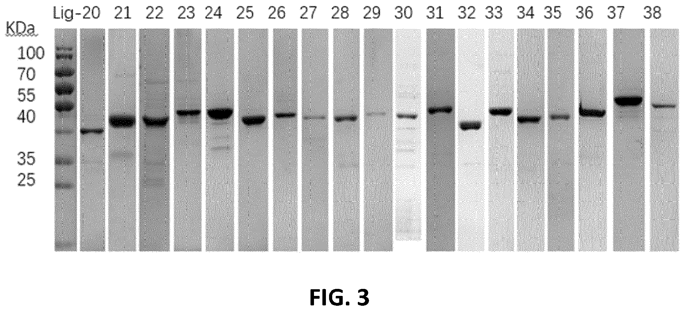

Genes of a ligase were constructed onto a pET28a vector; a histidine-tag was added at an N end of the ligase to facilitate the affinity chromatographic purification; the constructed recombinant plasmid was transformed into an *Escherichia coli* BL21(DE3) expression strain. The expression strain was inoculated into a 5 mL of LB medium (containing 50 µg/mL) in an inoculum size of 0.1%, and cultured at 37°C for 16 h. The obtained culture solution was transferred into 500 mL of LB medium in an inoculum size of 1%, and cultured at 37°C until OD = 0.6; 0.1 mM of IPTG was added for induction for 16 h at 20°C. The obtained induction culture solution was centrifuged at 4°C and 10,000 rpm for 20 min to obtain a bacterial sludge. The bacterial sludge was resuspended by a lysis buffer (50 mM Tris-Cl pH8.0, 0-500 mM NaCl, and 10% glycerol) such that the bacterium had a final concentration of 10%, and then ultrasonically crushed and centrifuged to obtain a crude enzyme solution. The crude enzyme solution was filtered with 0.22 µm of a filter membrane to obtain an enzyme solution; and the enzyme solution was purified with a nickel column, desalinated and subjected to liquid exchange, and secondary purification with a strong anionic column; the obtained enzyme solution was desalinated and subjected to liquid exchange, then stored into 30% glycerol. FIG. 1 shows a gel detection result of lig-20 purified by a nickel column; FIG. 2 shows a gel detection result of lig-20 purified by an ion column. The purification effect of the final ligase is shown in FIG. 3, indicating that purified enzymes are all purified by the purification modes of the present disclosure for catalytic reaction.

### Example 2

### Ligation and catalytic synthesis of double-stranded RNA

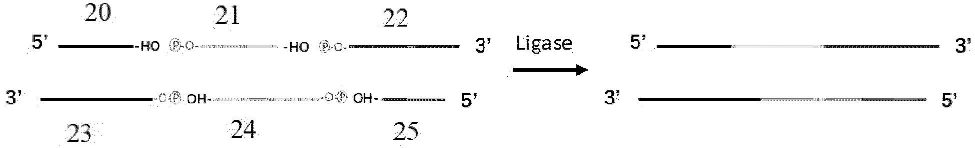

**Table 4**

| No. (SEQ ID NO.) | Sequence | 5' end | 3' end |
|---|---|---|---|
| 20 | UmGmAfGfUmGm | Hydroxy | Hydroxy |
| 21 | AfGfAmCmCfUfGmCm | Phosphate group | Hydroxy |
| 22 | GmAmCfUfGmCmAfCfUmAm | Phosphate group | Hydroxy |
| 23 | UfCmUmCfAfCmUmCfAfCmUm | Phosphate group | Hydroxy |
| 24 | AmGfUfCmGmCfAfGmGm | Phosphate group | Hydroxy |
| 25 | AmGfUfGmCm | Hydroxy | Hydroxy |

| | | | |
|---|---|---|---|
| Note: "m" denotes: 2' methoxy modification, and "f" denotes: 2' fluorine modification. | | | |

Substrates (SEQ ID NO.20, SEQ ID NO.21, SEQ ID NO.22, SEQ ID NO.23, SEQ ID NO.24, and SEQ ID NO.25) in Table 4 were prepared by a solid-phase synthesis method, i.e., a conventional chemical synthesis method.

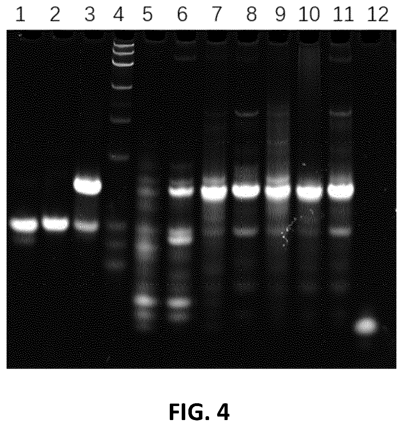

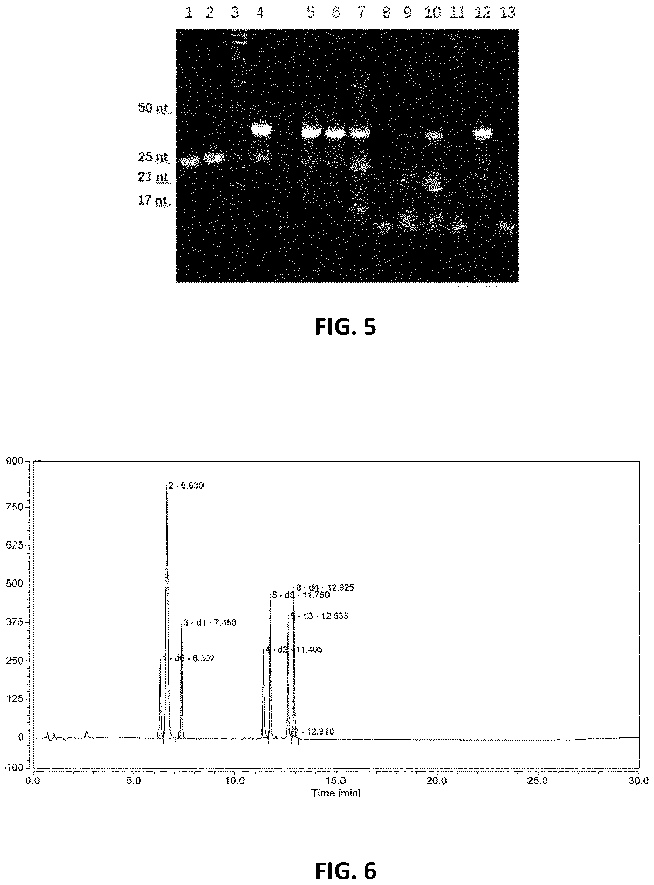

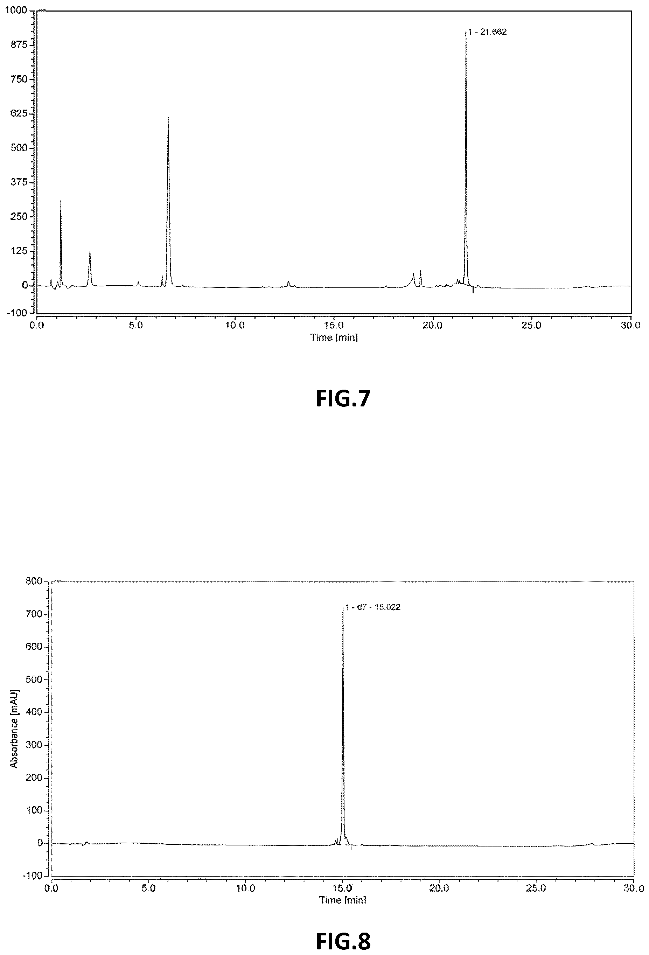

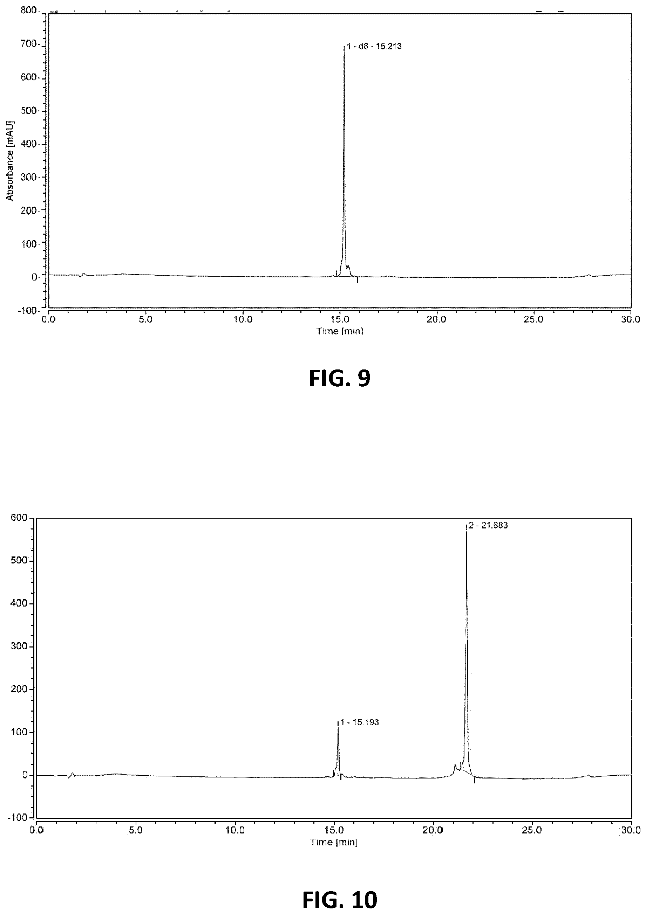

0.2 µmol (a final concentration of 400 µM) of 6 substrates in Table 4 were taken, respectively and added with 0.4 µmol of ATP and 10* ligation buffer (containing 50 mM tris-Cl, 10 mM MgCl₂, and1 mM DTT) such that the final volume was 500 µL. 0.1 mg of each lig-20, 21, 22, 23, 24, and 25 was added to different reaction tubes, respectively, and reacted for 3 h at 25°C, heated at 98°C for 2 min, and thermally insulated for 20 min at 80°C to deactivate the enzyme, and centrifuged to obtain supernatant. Denaturing polyacrylamide gel electrophoresis was adopted for analysis and detection. The results show the generation of product bands with high brightness, specifically as shown in FIG. 4 (1. A standard of the positive-sense strand product (ligated by SEQ ID NO.20, SEQ ID NO.21, and SEQ ID NO.22); 2. A standard of the antisense strand product (ligated by SEQ ID NO.23, SEQ ID NO.24, and SEQ ID NO.25); 3. Positive-sense strand + antisense strand; 4. Molecular weight marker of nucleic acid; 5. Reaction catalyzed by a ligase derived from *Trypanosoma brucei*; 6. Reaction catalyzed by a lig21; 7. Reaction catalyzed by a lig22; 8. Reaction catalyzed by a lig24; 9. Reaction catalyzed by a lig23; 10. Reaction catalyzed by a lig20; 11. Reaction catalyzed by a lig25; 12. Control group (containing 400 µM of a substrate, excluding an enzyme, a corresponding volume of 50 mM Tris-Cl buffer solution was added to replace the enzyme)), and FIG. 5 (1. Positive-sense strand; 2. Antisense strand; 3. Molecular weight marker of nucleic acid; 4. Positive-sense strand + antisense strand; 5. Reaction catalyzed by a lig35; 6. Reaction catalyzed by a lig31; 7. Reaction catalyzed by a lig34; 8. Reaction catalyzed by a lig1; 9. Reaction catalyzed by a lig15; 10. Reaction catalyzed by a lig37; 11. Reaction catalyzed by a lig16; 12. Reaction catalyzed by a lig32; 13. Control group (containing 400 µM of a substrate, excluding an enzyme, a corresponding volume of 50 mM Tris-Cl buffer solution was added to replace the enzyme). Samples were subjected to UPLC detection. The results are shown in FIG. 6 (control group including 400 µM of a substrate, excluding an enzyme, a corresponding volume of 50 mM Tris-Cl buffer solution was added to replace the enzyme; the solution was reacted for 3 h at 25°C, heated at 98°C for 2 min, thermally insulated for 20 min at 80°C, and centrifuged to obtain supernatant for UPLC analysis), and FIG. 7 (reaction catalyzed by a ligase lig24, and 21.662 min peak denotes the double-stranded product peak), FIG. 8 (appearance time of the positive-sense strand product is 15.022 min); FIG. 9 (appearance time of the antisense strand product is 15.213 min), and FIG. 10 (appearance time of the mixed solution of the positive-sense strand product and the positive-sense strand product, i.e., the double-stranded product, is 21.683 min; 15.192 min peak denotes the excessive amount of antisense strand product). The results show that the peak appearance position of the product is consistent with the standard, and there is basically no substrate remaining. After being identified by mass spectrometry (MS), the result of the positive-sense strand is 7,926.24 and its theoretical value is 7,925.98±7; the MS identification result of the antisense strand is 8,150.25, and its theoretical value is 8,152.07±8; the results are consistent with the standard. It indicates that the ligation reaction is catalyzed by lig-20, 21, 22, 23, 24, 25, 35, 31, 34, 37, and 32 to generate the ligated double-stranded RNA.

### Example 3

### Ligation and catalytic synthesis of double-stranded RNA

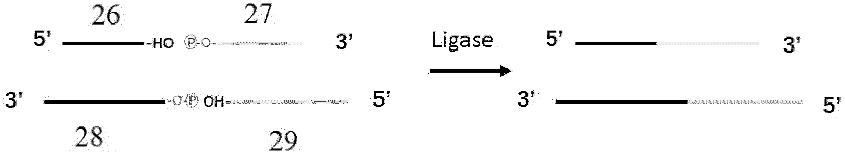

### Catalytic ligation of natural double-stranded RNA by a ligase

**Table 5**

| No. (SEQ ID NO.) | Sequence | 5' end | 3' end |
|---|---|---|---|
| 26 | UGAGUG | Hydroxy | Hydroxy |
| 27 | AGACCUGC | Phosphate group | Hydroxy |
| 28 | UCUCACUCACU | Phosphate group | Hydroxy |
| 29 | AGUCGCAGG | Hydroxy | Hydroxy |

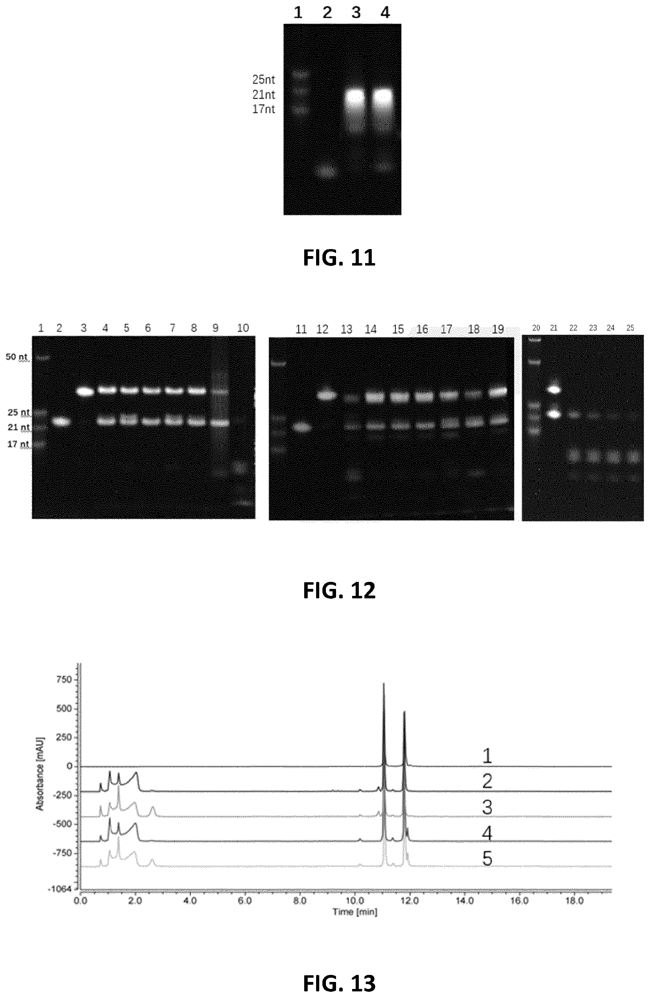

Substrates 26-29 were mixed in an equal proportion, and added with 10 µl of 1 mM ATP and 10* ligation buffer (containing 50 mM tris-Cl, 10 mM MgCl₂, and 1 mM DTT); 10 µL(2 mg/ml) of each pure enzyme lig20 and lig24 was added to different reaction tubes, respectively, and added with water such that the substrate concentration was 50 µM; the mixed solution was reacted for 3 h at 25°C, heated for 2 min at 98°C, and thermally insulated for 20 min at 80°C to deactivate the enzyme, and then centrifuged to obtain supernatant. After being identified by MS, the result of the positive-sense strand product (ligated by SEQ ID NO.26 and SEQ ID NO.27) was 4,488.15, and the result of the antisense strand product (ligated by SEQ ID NO.28 and SEQ ID NO.29) was 6,304.92; the theoretical value of the positive-sense strand was 4,485.73±4, and the theoretical value of the antisense strand was 6,302.79±6, and the generation of the product was thus determined. It indicates that the ligation reaction is catalyzed by lig20 and lig24 to generate the ligated double-stranded RNA. Results are shown in FIG. 11 (detection reaction results of SDS-PAGE diagram; lane 1 denotes the molecular weight marker; lane 2 denotes the negative control (the system contains a substrate, reaction buffer, ATP, exclusive of an enzyme, the enzyme is replaced by a corresponding volume of 50 mM tris-Cl); lanes 3 and 4 denote reaction systems after being catalyzed by the ligases).

### Example 4

### Ligation and catalytic synthesis of double-stranded RNA

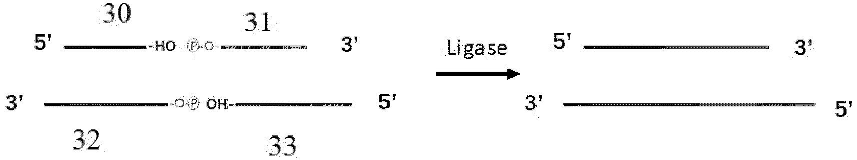

### Catalytic ligation of double strands of non-natural RNA by a ligase

**Table 6**

| No. (SEQ ID NO.) | Sequence | 5' end | 3' end |
|---|---|---|---|
| 30 | UmCmCmUmGfAmCfUfAmUmGfUm | Hydroxy | Hydroxy |
| 31 | AmUmUmGmAmUmUmAmUm | Phosphate group | GalNAc |
| 32 | CmGmsAmsAmsAfUmAmGmsUm | Phosphate group | Hydroxy |
| 33 | AmsAmAfUmUfsAmsAmCmUmsCmAmAmGmUf | Phosphate group | Hydroxy |

| | | | |
|---|---|---|---|
| Note: "m" denotes: 2' methoxy modification, and "f" denotes: 2' fluorine modification. "s" denotes: thiophosphate modification | | | |

Substrates 30-33 were mixed in an equal proportion, and added with 10 µl of 1 mM ATP and 10* ligation buffer (containing 50 mM tris-Cl, 10 mM MgCl₂, and 1 mM DTT); 10 µL(2 mg/ml) of each pure enzyme lig-20, 21, 22, 23, 24, 25, 31, 32, 33, 34, 35, 36, and 37 was added to different reaction tubes, respectively, and added with water such that the substrate concentration was 200 µM; in the meantime, an enzyme-free control reaction was set; the mixed solution was supplemented with 10 µL of 50 mM tris-Cl for reaction for 3 h 25°C, and heated for 2 min at 98°C, and thermally insulated for 20 min at 80°C to deactivate the enzyme, and then centrifuged to obtain supernatant. After being identified by MS, the result of the positive-sense strand product (ligated by SEQ ID NO.30 and SEQ ID NO.31) was 8,726.9, and the result of the antisense strand product (ligated by SEQ ID NO.32 and SEQ ID NO.33) was 7,765.9; the theoretical value of the positive-sense strand was 8,727.2±3, and the theoretical value of the antisense strand was 7,766.0±3. It indicates that the ligation reaction is catalyzed by ligases lig-20, 21, 22, 23, 24, 25, 31, 32, 33, 34, 35, 36, and 37 to generate the ligated double-stranded RNA. No product was generated to the lig-16, 17, 18, and 19. The SDS-PAGE diagram of FIG. 12 shows that the substrate was basically transformed completely to generate product bands.

In FIG. 12, lane 1: molecular weight standard of nucleic acid; lane 2: positive-sense strand product; lane 3: antisense strand product; lane 4: lig-20; lane 5: lig-21; lane 6: lig-22; lane 7: lig-23; lane 8: lig-24; lane 9: lig-25; lane 10: negative control (containing 200 µM substrate, a ligation buffer, ATP, without addition of an enzyme only, and the enzyme was replaced by an equal volume of 50 mM tris-Cl; the mixed solution was reacted for 3 h at 25°C, heated for 2 min at 98°C, and thermally insulated for 20 min at 80°C, and then centrifuged to obtain supernatant for gel running); lane 11: positive-sense strand product; lane 12: antisense strand product; lane 13: lig-37; lane 14: lig-33; lane 15: lig-36; lane 16: lig-32; lane 17: lig-31; lane 18: lig-35; and lane 19: lig-34. Lane 20: molecular weight standard of nucleic acid; lane 21: a mixed system of positive-sense and antisense strand products; lane 22: lig-16, lane 23: lig-17, lane 24: lig-1, and lane 25: lig-19.

By UPLC detection, the results show complete catalysis, as shown in FIG. 13 (ligation reaction catalyzed by ligases; 1 denotes the standard after mixing the positive-sense strand with the antisense strand; the two chains are separated in UPLC, consistent with the result of the SDS-PAGE diagram. 2-5 denote the reaction systems catalyzed by ligases, being lig-20, 21, 22, and 23, respectively).

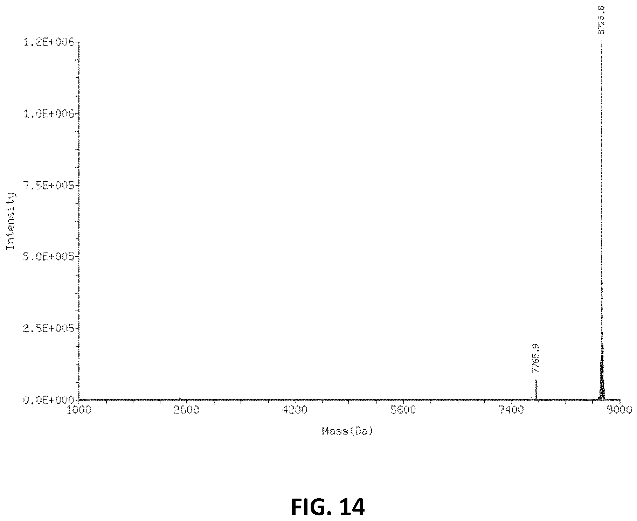

FIG. 14 shows the MS detection results: after reaction, the lig-20 reaction system was subjected to mass spectrometric detection; the molecular weight result conformed to the product molecular weight, indicating the generation of the ligated products.

The reaction was optimized; the reaction pH and reaction temperature were optimized; the result is that target products could be generated at Ph=6-8°C; the optimal pH is 7.5, and products could be generated at 0°C-40°C, and the optimal reaction temperature is 16°C. Results are shown in Table 7 below.

**Table 7**

| Catalytic activity | pH | | | | | Temperature | | | | |
|---|---|---|---|---|---|---|---|---|---|---|
| Ligase | 6.0 | 6.5 | 7.0 | 7.5 | 8.0 | 0 °C | 16 °C | 25 °C | 37 °C | 40 °C |
| lig-20 | + | + | ++ | ++ | + | ++ | ++ | ++ | + | + |
| lig-21 | ++ | ++ | ++ | ++ | + | ++ | ++ | ++ | ++ | ++ |
| lig-22 | ++ | ++ | ++ | ++ | ++ | ++ | +++ | ++ | + | + |
| lig-23 | + | ++ | ++ | ++ | + | ++ | ++ | ++ | + | + |
| lig-24 | + | ++ | ++ | ++ | + | ++ | +++ | ++ | ++ | + |
| lig-25 | ++ | ++ | ++ | +++ | + | ++ | +++ | +++ | ++ | ++ |

| | | | | | | | | | | |
|---|---|---|---|---|---|---|---|---|---|---|
| Note: + denotes an activity of 50%-60%; ++ denotes an activity of 60%-80%; and +++ denotes an activity of greater than 80%. | | | | | | | | | | |

### Example 5

### Ligation and catalytic synthesis of double-stranded RNA

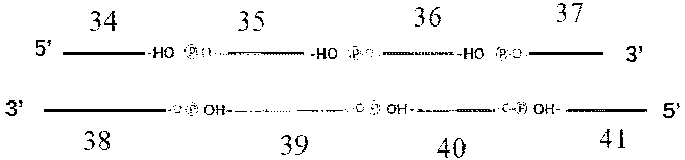

**Table 8**

| No. (SEQ ID NO.) | Sequence | 5' end | 3' end |
|---|---|---|---|
| 34 | GfUmGm | Hydroxy | Hydroxy |
| 35 | AfGfAmCmCfUfGmCm | Phosphate group | Hydroxy |
| 36 | GmAmCfUfGmCmAfCfUmAmAm | Phosphate group | Hydroxy |
| 37 | UfCmUmCfAfCmUm | Phosphate group | Hydroxy |
| 38 | UfCmUmCfAfCmUmCfAf | Phosphate group | Hydroxy |
| 39 | AmGfUfCmGmCfAfGmGm | Phosphate group | Hydroxy |
| 40 | AmGfUfGmCm | Phosphate group | Hydroxy |
| 41 | AmGmUmGmAmGmAmUmUm | Hydroxy | Hydroxy |

| | | | |
|---|---|---|---|
| Note: "m" denotes: 2' methoxy modification, and "f" denotes: 2' fluorine modification. | | | |

0.1 µmol (final concentration of 200 µM) of each substrate in Table 8 was taken and added with 20 µl of 10 mM ATP; 0.1 mg of each pure enzyme lig-20, 21, 22, 23, 24, and 25 was added to different reaction tubes, respectively, and added with 10* ligation buffer (containing 50 mM tris-Cl, 10 mM MgCl₂, and 1 mM DTT) such that the final volume was 0.5 mL; the mixed solution was reacted for 3 h at 25°C, heated for 2 min at 98°C, and thermally insulated for 20 min at 80°C to deactivate the enzyme, and then centrifuged to obtain supernatant. After being identified by MS, the result of the positive-sense strand product (ligated by SEQ ID NO.34, SEQ ID NO.35, SEQ ID NO.36, and SEQ ID NO.37) was 11,227.4, and the result of the antisense strand product (ligated by SEQ ID NO.38, SEQ ID NO.39, SEQ ID NO.40, and SEQ ID NO.41) was 10,583.7; the theoretical value of the positive-sense strand product was 11,225±8, and the theoretical value of the antisense strand product was 10,580±8, and the generation of the product was thus determined. It indicates that the ligation reaction is catalyzed by lig-20, 21, 22, 23, 24, and 25 to generate the ligated double-stranded RNA.

### Example 6

### Ligation and catalytic synthesis of long-chain RNA

**Table 9**

| No. (SEQ ID NO.) | Sequence | 5' end | 3' end |
|---|---|---|---|
| 20 | UmGmAfGfUmGm | Hydroxy | Hydroxy |
| 23 | UfCmUmCfAfCmUmCfAfCmUm | Phosphate group | Hydroxy |
| 42 | AGACCUGCGACUGCACUAUAGUUUACCAUGUGCA UCCGAGCUCAGCCCUUAAGCAUCAGCUGACUUAU CGUCGACUAACAGAUUAUUCGAGCUCCAUCUGCU UAAUCUUAGGCUUACCUCGAUUCUGCAAUCCAUU GUGCUACC | Phosphate group | Hydroxy |
| 43 | GGUAGCACAAUGGAUUGCAGAAUCGAGGUAAGCC UAAGAUUAAGCAGAUGGAGCUCGAAUAAUCUGUUA GUCGACGAUAAGUCAGCUGAUGCUUAAGGGCUGA GCUCGGAUGCACAUGGUAAACUAUAGUGCAGUCG CAGGUCU | Hydroxy | Hydroxy |

| | | | |
|---|---|---|---|
| Note: "m" denotes: 2' methoxy modification, and "f" denotes: 2' fluorine modification. | | | |

0.1 µmol of each substrate in Table 9 was taken and added with 20 µl of 10 mM ATP; 0.1 mg of each pure enzyme lig-21, 22, 23, 24, and 25 was added to different reaction tubes, respectively, and added with 10* ligation buffer (containing 50 mM tris-Cl, 10 mM MgCl₂, and 1 mM DTT) such that the final volume was 0.5 mL; the mixed solution was reacted for 3 h at 25°C, heated for 2 min at 98°C, and thermally insulated for 20 min at 80°C to deactivate the enzyme, and then centrifuged to obtain supernatant. After being identified by MS, the molecular weight of the positive-sense strand was 47,789.4, and the molecular weight of the antisense strand product was 50,130.2; the theoretical value of the positive-sense strand was 47,781.4±10, and the theoretical value of the antisense strand was 50,121±10, and the generation of the product was thus determined. It indicates that the ligation reaction is catalyzed by lig-21, 22, 23, 24, and 25 to generate the ligated double-stranded RNA.

As can be seen from the aforesaid description, the above examples of the present disclosure achieve the following technical effects: green and environmental protection; the present disclosure can be applied to specifically splice different nucleotide fragments, synthesize long-fragment nucleotide chains, and to catalyze the ligation between natural nucleotides and non-natural nucleotides, thereby solving the technical problem in the synthesis of nucleotide chains currently.

What is described above are merely preferred examples of the present disclosure and thus are not construed as limiting the present disclosure. There are various alterations and changes of the present disclosure to those skilled in the art. Any amendment, equivalent replacement, improvement, etc. made within the spirit and principle of the present disclosure shall fall within the protection scope of the present disclosure.

## Claims

1. A synthesis method for a double-stranded RNA, comprising the following steps:
S1. synthesizing single-stranded RNA fragments; and
S2. mixing the single-stranded RNA fragments, and linking the single-stranded RNA fragments with an RNA ligase to form the double-stranded RNA, wherein the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1 or Buttiauxella phage vB_ButM_GuL6.*

2. The synthesis method according to claim 1, wherein the single-stranded RNA fragments have a length of 3 to 200 nucleotides or analogues thereof.

3. The synthesis method according to claim 2, wherein the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.

4. The synthesis method according to claim 1, wherein the double-stranded RNA is natural RNA or non-natural RNA.

5. The synthesis method according to claim 4, wherein the non-natural RNA has a ribonucleotide with one or more of pentose ring 2'-position modification and 3'-position modification, phosphate α-position modification or base modification;
preferably, the pentose ring 2'-position modification comprises 2'-methoxy modification, 2'-fluoro modification, 2'-trifluoromethoxy modification, 2'-methoxyethyl modification, 2'-allyl modification, 2'-amino modification, or 2'-azido modification;
preferably, the phosphate α-position modification comprises phosphate α-position sulfur modification; and
preferably, the base modification comprises methylation modification and/or acetylation modification at any one or more of N1, N5, or N6 positions of a base.

6. The synthesis method according to claim 1, wherein the single-stranded RNA fragments are synthesized using a solid phase synthesis method.

7. The synthesis method according to claim 1, wherein the single-stranded RNA fragments comprise substrate 1 - substrate n of the single-stranded RNA fragments and substrate n+1 - substrate n+n of corresponding complementary single-stranded RNA fragments, wherein n ≥ 2,
there are complementary base pairs of ≥ 3 nucleotides between the complementary single-stranded RNA fragments;
there are base complementary pairs of ≥ 2 nucleotides at the 5'-ends of the substrate 2 and the substrate n+1, and the substrate n and the substrate n+n-1;
optionally, 5'- and 3'-ends outside the substrate 1 and the substrate n+1 are linked by chemical bonds; and
optionally, 5'- and 3'-ends outside the substrate n and the substrate n+n are linked by chemical bonds.

8. The synthesis method according to claim 1, wherein the reaction temperature of S2 is 0°C to 60°C, preferably 4°C to 37°C, the reaction time is 0.5 h to 24 h, and the pH is 6 to 8.5.

9. Use of an RNA ligase in the synthesis of double-stranded RNA, wherein the RNA ligase is an RNA ligase derived from *Vibrio phage, Escherichia phage, Klebsiella phage, Bacteriophage, thermophilic bacteriophage, Acidobacteriabacterium, Salmonellaenterica, YersiniaphagevB_YepM_ZN18, Shigella phage pSs-1 or Buttiauxella phage vB_ButM_GuL6.*

10. The synthesis method according to claim 9, wherein the RNA ligase derived from *Vibrio phage* has an amino acid sequence as shown in SEQ ID NO: 1, 2, 5, 6, 13, 14, 17 or 19; the RNA ligase derived from *Escherichia phage* has an amino acid sequence as shown in SEQ ID NO: 3 or 15; the RNA ligase derived from *Klebsiella phage* has an amino acid sequence as shown in SEQ ID NO: 4 or 16; the RNA ligase derived from *Acidobacteriabacterium* has an amino acid sequence as shown in SEQ ID NO: 7; the RNA ligase derived from *Salmonellaenterica* has an amino acid sequence as shown in SEQ ID NO: 8; the RNA ligase derived from *YersiniaphagevB_YepM_ZN18* has an amino acid sequence as shown in SEQ ID NO: 9; the RNA ligase derived from *Shigella phage pSs-1* has an amino acid sequence as shown in SEQ ID NO: 10; the RNA ligase derived from *Buttiauxella phage vB_ButM_GuL6* has an amino acid sequence as shown in SEQ ID NO: 11; the RNA ligase derived from *Bacteriophage* has an amino acid sequence as shown in SEQ ID NO: 12; and the RNA ligase derived from the *thermophilic Bacteriophage* has an amino acid sequence as shown in SEQ ID NO: 18.
